# EUROPÄISCHE PATENTSCHRIFT

(11) **EP 1 230 509 B1**
(45) Veröffentlichungstag und Bekanntmachung des Hinweises auf die Patenterteilung: **25.05.2005**
(21) Anmeldenummer: 00977533.9
(22) Anmeldetag: 13.11.2000
(51) Int. Cl.: F16N 29/02, G01N 27/22, G01N 21/43

(54) **VERFAHREN UND VORRICHTUNG ZUR ÜBERWACHUNG DES ÖLSTROMS EINER EINRICHTUNG ZUR ÖL + LUFT-SCHMIERUNG VON BAUTEILEN**
METHOD AND DEVICE FOR MONITORING THE FLOW OF OIL PERTAINING TO A DEVICE FOR THE OIL AND AIR LUBRICATION OF COMPONENTS
PROCEDE ET DISPOSITIF POUR SURVEILLER LE FLUX D'HUILE D'UN DISPOSITIF SERVANT A LA LUBRIFICATION PAR HUILE ET AIR DE COMPOSANTS

(30) Priorität: 16.11.1999 DE 19956958
(43) Veröffentlichungstag der Anmeldung: 14.08.2002
(73) Patentinhaber: Willy Vogel Aktiengesellschaft, 12277 Berlin (DE)
(72) Erfinder: GACA, Hans, 63075 Offenbach/Main (DE); SPIESS, Götz, 14089 Berlin (DE)
(74) Vertreter: Grünecker, Kinkeldey, Stockmair & Schwanhäusser Anwaltssozietät
(86) Internationale Anmeldenummer: PCT/EP2000/011202
(87) Internationale Veröffentlichungsnummer: WO 2001/036861

(56) Entgegenhaltungen:
- EP-A- 0 924 509
- US-A- 4 210 809
- US-A- 4 346 786
- US-A- 4 599 888
- US-A- 4 713 603
- US-A- 5 083 862
- US-A- 5 466 946
- PATENT ABSTRACTS OF JAPAN vol. 015, no. 032 (M-1073), 25. Januar 1991 (1991-01-25) & JP 02 271197 A (NIPPON SEIKO KK), 6. November 1990 (1990-11-06)

## Beschreibung

Die Erfindung betrifft ein Verfahren zur Überwachung eines Ölstroms bei einer Einrichtung zur Öl+Luft-Schmierung von Aggregaten oder deren Lager, bei dem Öltropfen durch einen Luftstrom entlang einer Wandung eines Rohres transportiert und dabei schlierenartig auseinander gezogen werden, wobei Schwankungen des Ölstroms erfasst werden und ein den Schwankungen entsprechendes Signal ausgegeben wird und wobei die Schwankungen des Ölstroms durch einen Schlierensensor erfasst werden, der eine Schlierenform, Schlierengröße, Schlierenfrequenz und/oder Schlierenfolge des Ölstroms an der Wandung des Rohres auf optoelektronischem Wege erfasst.

Die sogenannte Öl+Luft-Schmierung stellt eine rationelle Schmierungsmethode dar, deren Anwendung sich immer dann anbietet, wenn ein fein dosierter Ölstrom möglichst kontinuierlich einer Reibstelle zugeführt werden soll. Letzteres ist beispielsweise bei schnelllaufenden Wälzlagem der Fall, wie man ihnen u. a. in Spindeln von Werkzeugmaschinen begegnet. Die Überwachung des Ölstroms oblag bisher dem Bedienungspersonal, das sie durch unmittelbare Inaugenscheinnahme vornahm. (Betriebshandbuch: Schmierungstechnik Zentralschmieranlagen der Willy Vogel AG, 1996, S. 4.15).

Es versteht sich, dass die vorgenannte Art der Überwachung nicht nur die Verwendung durchsichtiger Rohrleitungen, sondern auch eine hinreichende Aufmerksamkeit der jeweiligen Bedienungsperson voraussetzte.

Aus der DE 44 39 380 A1 ist eine Schmiereinrichtung bekannt, bei der Öl von einem fließenden Film durch Luftströmung in kleine Tröpfchenpartikel abgelöst und einer Schmierstelle zugeführt wird. Dabei soll eine Ölvemebelung vermieden werden. Eine dynamische Messung der Öl-Durchflussmenge ist mit einem Lichtsensor bei der Vorrichtung der DE 44 39 380 A1 möglich.

Auch bei der Vorrichtung der JP 02 271 197 wird ein Ölfilm an einer Wand mittels einer Luftströmung transportiert und durch Infrarotsensoren überwacht.

Der Erfindung liegt die Aufgabe zugrunde, ein Verfahren der in Betracht gezogenen Art zu schaffen, das eine automatische Überwachung der Schmiermittelversorgung einer Lagerstelle mit dem Charakter der Öl-Luft-Schmierung Rechnung tragenden Mitteln ermöglicht.

Diese Aufgabe wird erfindungsgemäß dadurch gelöst, dass Schwankungen des Ölstroms durch einen Schlierensensor erfasst werden und vom Schlierensensor ein den Schwankungen entsprechendes Signal ausgegeben wird.

Das erfindungsgemäße Verfahren macht sich die wellenartige Wanderbewegung der Ölschlieren zunutze, um vorzugsweise unter Erfassung der Frequenz der Wellenberge bzw. Wellentäler entweder auf den Schmierprozess Einfluss zu nehmen oder aber das zu schmierende Aggregat im Falle einer Unterversorgung mit Schmiermitteln bzw. bei einem Ausfall der Schmiermittelversorgung abzuschalten.

Gegenstand der Erfindung ist außerdem eine Vorrichtung zur Überwachung des Ölstroms einer Einrichtung zur Öl+Luft-Schmierung von Bauteilen.

Die Erfindung wird im Folgenden anhand der in der beigefügten Zeichnung dargestellten Biockschaubilder näher erläutert. Es zeigen:
- Fig 1: die schematische Darstellung einer auf kapazitiver Basis arbeitenden Überwachungsvorrichtung, die nicht durch die Ansprüche geschützt ist, und
- Fig. 2: die schematische Darstellung einer auf optoelektronischer Basis arbeitenden Überwachungsvorrichtung gemäß der Erfindung.

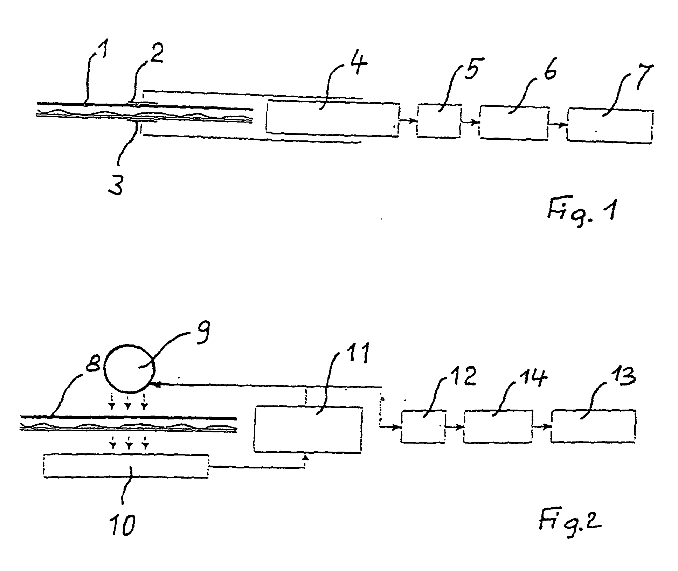

In Fig. 1 ist 1 ein verhältnismäßig enges, in der Praxis etwa 4 mm starkes Kunststoffrohr, das mindestens 1 m lang sein sollte, um vor der Überwachungsstelle die gewünschte Schlierenbildung des sich nicht mit der Transportluft vermischenden Ölfilms sicherzustellen. An sich gegenüberliegenden Seiten des Rohrs 1 sind zwei das Rohr 1 jeweils partiell umschließende, der Wandform angepasste Elektroden 2 und 3 angeordnet, die die Platten eines Kondensators bilden, dessen Feld durch die Folge, Form und Größe der Schlieren des Ölstroms beeinflusst wird und dessen Kapazitätsänderungen durch eine Art Elektrometer 4 erfasst und über einen Filter 5 und ein Zeitglied 6 der Schaltstufe 7 einer Steuereinheit zugeleitet werden, die entweder die in das Rohr 1 eingeleitete Ölmenge bzw. die Stärke des Luftstroms verändert oder aber ein zu schmierendes Aggregat abschaltet.

Eine zweite Überwachungsmöglichkeit bietet die in Fig. 2 schematisch dargestellte Lösung, bei der es sich im Gegensatz zum kapazitiven Schlierensensor gemäß Figur 1 um einen optoelektronischen Schlierensensor handelt.

In Fig. 2 ist 8 ein aus durchsichtigem Kunststoff bestehendes Rohr, das im Bereich einer mindestens 1 m weit von der Einspeisungsstelle des Öl+Luftstroms gelegenen Überwachungszone eine Lichtquelle 9 und einen Empfänger 10 für das von der Lichtquelle 9 ausgestrahlte Licht aufweist. Um einen Fremdlichteinfluss zu unterdrücken, wird die Intensität des dem Empfänger 10 zugeführten Lichts mit Hilfe eines Reglers 11 konstant gehalten, d. h. die Strahlungsintensität der Lichtquelle wird im Takt der Schlieren so geregelt, dass der Empfänger 10 trotz der durch die Schlieren an sich bedingten schwankenden Dämpfung des Lichts stets mit der gleichen Lichtmenge beaufschlagt wird. Die die Lichtquelle 9 steuernden pulsierenden Signale sind somit ein Indikator für das Vorhandensein von Ölschlieren.

Ein dem Regler 11 nachgeschaltetes, als Bandpassfilter ausgebildetes Filter 12 unterdrückt wiederum untypische Signale, wie sie in diesem Fall beispielsweise durch Veränderungen des Sonnenlichts hervorgerufen werden können. Das der Schaltstufe 13 vorgeschaltete Zeitglied 14 erfüllt die gleiche Funktion wie das Zeitglied 6.

## Patentansprüche

1. Verfahren zur Überwachung eines Ölstroms bei einer Einrichtung zur Öl+Luft-Schmierung von Aggregaten oder deren Lager, bei dem Öltropfen durch einen Luftstrom entlang einer Wandung eines Rohres (1) transportiert und dabei schlierenartig auseinander gezogen werden, wobei Schwankungen des Ölstroms erfasst werden und ein den Schwankungen entsprechendes Signal ausgegeben wird und wobei die Schwankungen des Ölstroms durch einen Schlierensensor (2, 3, 4) erfasst werden, der eine Schlierenform, Schlierengröße, Schlierenfrequenz und/oder Schlierenfolge des Ölstroms an der Wandung des Rohres auf optoelektronischem Wege erfasst, **dadurch gekennzeichnet, dass** die Strahlungsintensität einer Lichtquelle (9) durch einen Regler (11) im Takt der Schlieren so geregelt wird, dass ein Empfänger (10) stets mit der gleichen Lichtmenge beaufschlagt wird.

2. Verfahren nach Anspruch 1 oder 2, **dadurch gekennzeichnet, dass** das Signal des Schlierensensors zur Erzeugung einer Anzeige verwendet wird.

3. Verfahren nach einem der oben genannten Ansprüche, **dadurch gekennzeichnet, dass** das Signal des Schlierensensors zur Veränderung der in das Rohr eingeleiteten Ölmenge verwendet wird.

4. Verfahren nach einem der oben genannten Ansprüche, **dadurch gekennzeichnet, dass** das Signal des Schlierensensors zur Veränderung der Stärke des in das Rohr eingeleiteten Luftstroms verwendet wird.

5. Verfahren nach einem der oben genannten Ansprüche, **dadurch gekennzeichnet, dass** untypische Signale des Schlierensensors durch einen Filter unterdrückt werden.

6. Vorrichtung zur Überwachung eines Ölstroms bei einer Einrichtung zur Öl+Luft-Schmierung von Aggregaten oder deren Lager, mit einem abschnittsweise lichtdurchlässigen Rohr (1), durch das ein Luftstrom gerichtet ist und entlang dessen Wandung sich schlierenartig auseinandergezogene Öltropfen bilden, wobei am Rohr ein Sensor (2, 3, 4) angeordnet ist, durch den Schwankungen des Ölstroms erfassbar sind und ein den Schwankungen des Ölstroms entsprechendes Signal an eine Auswerteeinheit ausgebbar ist, und wobei der Sensor als ein Schlierensensor (2, 3, 4) ausgebildet ist, durch den eine Schlierenform, Schlierengröße, Schlierenfrequenz und/oder Schlierenfolge erfassbar ist und der einen Empfänger und eine auf den Empfänger gerichtete Lichtquelle (9) umfasst, die im Bereich des lichtdurchlässigen Abschnittes des Rohrs angeordnet sind, **dadurch gekennzeichnet, dass** der Schlierensensor (2, 3, 4) einen Regler umfasst, durch den die Lichtintensität der Lichtquelle so regelbar ist, dass der Empfänger (10) im Betrieb stets mit der gleichen Lichtmenge beaufschlagt ist, und durch den das Signal an die Auswerteeinheit erzeugbar ist.

## Claims

1. A method of monitoring an oil flow in a means for the oil and air lubrication of units or the bearings thereof, in which oil drops are transported by an air flow along a wall of a tube (1) and are thereby expanded in a streak-like manner, wherein fluctuations of the oil flow are detected and a signal that corresponds to the fluctuations is output, and wherein the fluctuations of the oil flow are detected by a streak sensor (2, 3, 4), which opto-electronically detects a streak shape, streak size, streak frequency and/or streak sequence of the oil flow on the wall of the tube, **characterized in that** the radiation intensity of a light source (9) is controlled by a controller (11) in the rhythm of the streaks in a manner that a receiver (10) is permanently biased by the same amount of light.

2. A method as claimed in claim 1 or 2, **characterized in that** the signal of the streak sensor is used for generating a display.

3. A method as claimed in one of the above-mentioned claims, **characterized in that** the switching unit changes an oil quantity introduced into the tube according to the pulsating signal from the controller.

4. A method as claimed in one of the preceding claims, **characterized in that** the signal of the streak sensor is used for varying a strength of the air flow introduced into the tube.

5. A method as claimed in one of the preceding claims, **characterized in that** untypical signals of the streak sensor are suppressed by a filter.

6. A device for monitoring an oil flow in a means for the oil and air lubrication of units and the bearings thereof, comprising a tube (1) that has transparent sections, through which an air flow is directed and along whose wall oil drops expanded in a streak-like manner are formed, wherein a sensor (2, 3, 4) is arranged on the tube, through which fluctuations of the oil flow can be detected and a signal corresponding to the fluctuations of the oil flow can be output to an evaluation unit, and wherein the sensor is formed as a streak sensor (2, 3, 4) through which streak shape, streak size, streak frequency and/or streak sequence can be detected and which comprises a receiver and a light source directed towards the receiver, which are arranged in the area of the transparent section of the tube, **characterized in that** the streak sensor (2, 3, 4) comprises a controller through which the light intensity of the light source can be controlled in a manner that the receiver (10) during operation can permanently be biased with the same amount of light and through which a signal to the evaluation unit can be generated.

## Revendications

1. Procédé pour surveiller un flux d'huile dans une installation pour la lubrification huile+air de groupes de machines ou de leurs paliers, dans lequel des gouttes d'huile sont transportées par un flux d'air le long d'une paroi d'un tuyau (1) et y sont étirées sous forme de stries, dans lequel des fluctuations du flux d'huile sont détectées et un signal correspondant aux fluctuations est émis, et dans lequel les fluctuations du flux d'huile sont détectées par un capteur strioscopique (2, 3, 4) qui détecte par voie optoélectronique une forme de strie, une grandeur de strie, une fréquence de stries et/ou une succession de stries du flux d'huile sur la paroi du tuyau, **caractérisé en ce que** l'intensité de rayonnement d'une source lumineuse (9) est régulée à la cadence des stries par un régulateur (11) de telle manière qu'un récepteur (10) est toujours exposé à la même quantité de lumière.

2. Procédé selon la revendication 1 ou 2, **caractérisé en ce que** le signal du capteur strioscopique est utilisé pour produire une indication optique.

3. Procédé selon l'une quelconque des revendications susmentionnées, **caractérisé en ce que** le signal du capteur strioscopique est utilisé pour modifier une quantité d'huile introduite dans le tuyau.

4. Procédé selon l'une quelconque des revendications susmentionnées, **caractérisé en ce que** le signal du capteur strioscopique est utilisé pour modifier la force du flux d'air introduit dans le tuyau.

5. Procédé selon l'une quelconque des revendications susmentionnées, **caractérisé en ce que** des signaux non typiques du capteur strioscopique sont éliminés par un filtre.

6. Dispositif pour surveiller un flux d'huile dans une installation pour la lubrification huile+air de groupes de machines ou de leurs paliers, comprenant un tuyau (1) à segments transparents, qui est traversé par un flux d'air et le long de la paroi duquel se forment des gouttes d'huile étirées en stries, dans lequel un capteur (2, 3, 4) est disposé sur le tuyau, lequel capteur permet de détecter des fluctuations du flux d'huile et d'émettre un signal correspondant aux fluctuations du flux d'huile vers une unité d'évaluation, et dans lequel le capteur est formé comme un capteur strioscopique (2, 3, 4), lequel permet de détecter une forme de strie, une grandeur de strie, une fréquence de stries et/ou une succession de stries et comprend un récepteur et une source lumineuse (9) dirigée vers le récepteur, lesquels sont disposés dans la zone du segment transparent du tuyau, **caractérisé en ce que** le capteur strioscopique (2, 3, 4) comprend un régulateur qui permet de réguler l'intensité lumineuse de la source lumineuse de telle manière que le récepteur (10) en fonctionnement est toujours exposé à la même quantité de lumière, et qui permet d'émettre un signal vers l'unité d'évaluation.
